(19) **Europäisches Patentamt / European Patent Office / Office européen des brevets**

(11) **EP 2 493 525 B2**

(12) **NOUVEAU FASCICULE DE BREVET EUROPEEN**
Après la procédure d'opposition

(45) Date de publication et mention de la décision concernant l'opposition:
**04.01.2023 Bulletin 2023/01**

(45) Mention de la délivrance du brevet:
**08.02.2017 Bulletin 2017/06**

(21) Numéro de dépôt: **10785127.1**

(22) Date de dépôt: **15.10.2010**

(51) Classification Internationale des Brevets (IPC):
*A61M 1/16* $^{(2006.01)}$

(52) Classification Coopérative des Brevets (CPC):
**A61M 1/16; A61M 1/1609; A61M 1/1613; A61M 1/1619**

(86) Numéro de dépôt international:
**PCT/FR2010/052201**

(87) Numéro de publication internationale:
**WO 2011/051593 (05.05.2011 Gazette 2011/18)**

(54) **DISPOSITIF ET PROCEDE DE CARACTERISATION D'UN TRAITEMENT EXTRACORPOREL DU SANG, ET APPAREIL DE TRAITEMENT EXTRACORPOREL METTANT EN OEUVRE UN TEL DISPOSITIF**

VORRICHTUNG UND VERFAHREN ZUR CHARAKTERISIERUNG EINER EXTRAKORPORALEN BLUTBEHANDLUNG UND MASCHINE FÜR EXTRAKORPORALE BLUTBEHANDLUNG MIT EINER DERARTIGEN VORRICHTUNG

DEVICE AND METHOD FOR CHARACTERIZING AN EXTRACORPOREAL BLOOD TREATMENT, AND EXTRACORPOREAL TREATMENT APPARATUS IMPLEMENTING SUCH A DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **30.10.2009 FR 0957693**

(43) Date de publication de la demande:
**05.09.2012 Bulletin 2012/36**

(73) Titulaire: **RD Nephrologie**
**34000 Montpellier (FR)**

(72) Inventeurs:
- **ARGILES CISCART, Angel**
  **F-34980 Saint Gely du Fesc (FR)**
- **FICHEUX, Alain**
  **F-34790 Grabels (FR)**

(74) Mandataire: **IPAZ**
**Bâtiment Platon**
**Parc Les Algorithmes**
**91190 Saint-Aubin (FR)**

(56) Documents cités:

| | |
|---|---|
| EP-B1- 0 166 920 | DE-A1- 2 624 384 |
| FR-A1- 2 696 644 | FR-A1- 2 739 780 |
| US-A- 5 662 806 | US-A- 5 725 773 |
| US-A1- 2003 216 677 | US-A1- 2003 216 677 |
| US-A1- 2003 231 294 | |

- **ARGILES et al.: "Precise quantification of dialysis using continuous sampling of spent dialysate and total dialysate volume measurement", Kidney International, vol. 52, 1997, pages 530-537,**

EP 2 493 525 B2

**Description**

L'invention porte sur un dispositif et un procédé de caractérisation d'un traitement extracorporel du sang réalisé par un appareil de traitement extracorporel. L'invention concerne également une utilisation de ce dispositif et un appareil de traitement extracorporel du sang mettant en oeuvre un tel dispositif.

L'invention porte plus particulièrement sur un dispositif permettant de déterminer un ou plusieurs paramètres cliniques d'efficacité relatifs au traitement extracorporel du sang réalisé par un appareil de traitement extracorporel rejetant un liquide de traitement extracorporel, qui sera appelé « filtrat » dans la suite de la description.

Par « caractérisation » on entend une évaluation ou une quantification du traitement extracorporel réalisé par l'appareil de traitement extracorporel.

Un traitement extracorporel du sang peut par exemple être une dialyse, une ultrafiltration pure, une hémodialyse, une hémofiltration, une hémodiafiltration ou encore une dialyse péritonéale.

La dialyse, par exemple, est une technique de purification de solutions faisant intervenir la diffusion par gradient de concentration entre deux liquides. La dialyse est réalisée par l'intermédiaire d'un liquide, appelé dialysat, qui après le traitement est appelé dialysat usé. Le dialysat usé est rejeté à l'égout.

Actuellement, pour caractériser un traitement extracorporel du sang, il existe trois méthodes :

- par dosage de prélèvements de sang avant et après une séance. Cette méthode présente une source d'erreur liée principalement aux difficultés dans l'établissement du volume de distribution dans l'organisme des solutés épurés en cours de séance et des multiples facteurs modulant les transferts entre les différents secteurs d'un organisme vivant (intracellulaire, extracellulaire, intra vasculaire,...), ainsi que leur modification par la séance de dialyse. Les prélèvements de sang doivent également être espacés pour des patients souvent anémiés ;
- par mesure d'efficacité instantanée (clairance ou dialysance). Cette méthode est aussi soumise à des erreurs car la mesure d'efficacité instantanée représente un instant T et doit être extrapolée pour obtenir une valeur moyenne. De plus, cette méthode nécessite une estimation de la masse initiale de soluté à épurer ;
- par la collection totale du filtrat, c'est-à-dire du liquide de traitement rejeté par l'appareil de traitement extracorporel. La concentration du soluté étudié multiplié par le volume recueilli donnera la masse totale de soluté extrait.

Cette dernière méthode est la méthode de référence car elle est fiable et tient compte des modifications en cours de séance.

Cependant, cette méthode est basée sur l'analyse de la totalité du filtrat. Elle ne peut donc être mise en oeuvre en routine car la quantité de filtrat est importante. Par exemple, la quantité totale de filtrat lors d'une séance de traitement extracorporel type hémodialyse est en général supérieure à 100 litres.

Par ailleurs, cette technique de caractérisation d'un traitement extracorporel du sang basée sur l'utilisation de la totalité du filtrat rejeté lors d'une séance de traitement ne peut pas être mise en oeuvre dans une clinique du fait de la quantité importante de filtrat.

De plus, la collection et le stockage de la totalité du filtrat dans un réceptacle d'évacuation représente un risque de contamination dû à la stagnation du filtrat dans un réceptacle ouvert.

Il a été montré, notamment par les inventeurs de la présente invention, que la caractérisation d'une dialyse pouvait être effectuée en se basant sur une partie du filtrat par la technique dite de « Continuous Sampling of Spent Dialysate » (CSSD) : Argiles, A, Ficheux, A, Thomas, A, et al: Precise quantification of dialysis using Continuous Sampling of Spent Dialysate and total dialysate volume measurement. Kidney Int 1997 52: 530-537.

Il n'existe actuellement aucun appareil permettant de caractériser, in situ, un traitement extracorporel du sang. Les praticiens néphrologues doivent en fait attendre que les résultats d'analyse du filtrat reviennent de laboratoires extérieurs au service ou centre de traitement.

Un but de l'invention est de remédier aux inconvénients précités.

Un but de l'invention est de proposer un appareil pour la caractérisation d'un traitement extracorporel du sang ne nécessitant pas d'analyses en laboratoire ou d'analyses sanguines.

Enfin, un autre but de l'invention est de proposer un dispositif pour la caractérisation d'un traitement extracorporel du sang peu couteux, compact, et pouvant être intégré dans des appareils de traitement existants.

L'invention est caractérisée dans les revendications. Par « filtrat » on désigne le liquide sortant de l'appareil de traitement extracorporel tout au long de la séance de traitement extracorporel du sang. Ce liquide est par exemple constitué de dialysat usé dans le cas d'une dialyse mettant en oeuvre un dialysat, ou simplement d'ultrafiltrat de plasma quand il n'y a pas de liquide de dialyse qui entre au contact du sang dans le cas d'une ultrafiltration et d'une hémofiltration.

Le dispositif selon l'invention base la caractérisation d'une dialyse, et plus généralement d'un traitement extracorporel réalisé par un appareil de traitement extracorporel, sur un échantillon de ce filtrat rejeté par l'appareil de traitement et prélevé en continu lors du traitement. Ainsi, le dispositif selon l'invention permet de s'affranchir du stockage et de l'utilisation de la totalité du filtrat et du volume important que représente la totalité de ce filtrat.

Ainsi, le dispositif selon l'invention peut présenter de faibles dimensions et peut être utilisé in situ, dans les cliniques, pour la caractérisation in situ d'un traitement réalisé.

De plus, le dispositif selon l'invention s'affranchie des problèmes de contamination due à la collecte de la totalité du filtrat.

Avantageusement, le dispositif selon l'invention permet de réaliser une caractérisation d'un traitement extracorporel du sang basée entièrement sur une partie du filtrat et ne nécessite pas d'analyse en laboratoire.

Le dispositif selon l'invention ne nécessite aucune intervention de la part d'un opérateur pour son fonctionnement et peut être totalement automatisé.

Avantageusement, les moyens de détermination peuvent comprendre des moyens de mélange d'au moins une partie du filtrat avec au moins un réactif et/ou au moins un indicateur.

Dans un exemple particulier d'application non limitatif, le paramètre recherché est le taux d'urée extraite par une dialyse, le réactif utilisé peut être l'uréase utilisé en combinaison avec un indicateur qui est l'indophénol.

Les moyens de prélèvement réalisent un prélèvement en continu du filtrat lors du traitement extracorporel du sang. Ainsi, une petite quantité de filtrat est prélevée en continu.

Le dispositif selon l'invention peut en outre comprendre des moyens d'échantillonnage pour prélever une quantité prédéterminée de filtrat, dit échantillon instantané, à un instant donné lors du traitement. En effet, le dispositif selon l'invention peut comprendre des moyens pour la détermination d'une valeur instantanée d'un paramètre en réalisant une analyse d'un échantillon de filtrat prélevé de manière instantanée à un instant $t_i$ lors du traitement. Un tel prélèvement instantané peut être réalisé à tout instant du traitement.

Les moyens d'échantillonnage peuvent être directement ou indirectement reliés aux moyens de détermination, lesdits moyens de détermination réalisant la détermination d'une valeur instantanée, audit instant donné, d'au moins un paramètre par analyse dudit échantillon instantané.

Dans un exemple particulier d'application, les moyens d'échantillonnage peuvent être agencés pour un prélèvement instantané en début de traitement pour déterminer par exemple le taux de potassium dans le sang. La valeur du taux de potassium peut ensuite être utilisée pour la prescription du traitement : durée de dialyse ou valeur des débits de sang et dialysat, concentration de potassium ou autres solutés dans le filtrat pour le traitement en cours, ou encore le type d'appareil de traitement à prescrire pour les traitements suivants.

Selon une version particulière du dispositif selon l'invention, les moyens de mélange peuvent comprendre, au moins en partie, les moyens d'échantillonnage. En effet, les moyens d'échantillonnage peuvent correspondre au moins en partie, aux moyens de mélange.

Avantageusement, les moyens de prélèvement peuvent être agencés pour prélever un échantillon inférieur à 50ml de filtrat sur toute la durée du traitement. Une telle quantité de 50ml représente une quantité très faible par rapport à la quantité totale de plus de 100 litres de filtrat.

Par ailleurs, les moyens de prélèvement peuvent être reliés au circuit d'évacuation par un premier circuit comportant au moins une vanne, ladite vanne étant :

- ouverte lors du prélèvement, et
- fermée lors de l'analyse du filtrat prélevé.

Il est également possible d'utiliser un clapet anti retour à la place de chaque vanne, le clapet anti-retour étant disposé de sorte que le sens passant est vers les moyens de prélèvement.

En particulier, les moyens de prélèvement peuvent comprendre au moins une seringue reliée, au moins indirectement, au circuit d'évacuation et actionnée de manière continue pour prélever le filtrat à un débit sensiblement constant depuis ledit circuit d'évacuation.

Les moyens de prélèvement peuvent comprendre au moins une cuve cylindrique ou une chambre, munie d'un piston permettant d'aspirer ou de refouler un liquide sur le même principe qu'une seringue.

Plus généralement, les moyens de prélèvement peuvent comprendre un réceptacle muni de moyens permettant de remplir et de vider ce réceptacle.

D'une manière similaire, les moyens d'échantillonnage peuvent comprendre une seringue reliée au circuit d'évacuation, une cuve cylindrique ou une chambre muni d'un piston ou tout autre réceptacle muni de moyens permettant de le remplir et de le vider.

A chaque seringue peut être associé au moins un moteur pour actionner la seringue dans les deux sens. Par exemple, la seringue des moyens de prélèvement est actionnée de manière continue lors du traitement, dans le sens du remplissage de la seringue. Avant une analyse la même seringue est actionnée dans le sens du vidage de la seringue pour envoyer au moins une partie du filtrat prélevé vers les moyens de détermination en vue de son analyse.

Les moyens de détermination peuvent comprendre un photomètre ou une cellule de mesure par spectroscopie. Les moyens de détermination peuvent comprendre tous types de cellules de mesure connues.

Selon un exemple de réalisation du dispositif selon l'invention ;

- les moyens de prélèvement sont reliés au conduit d'évacuation par un premier circuit comportant une première vanne ouverte lors du prélèvement et fermée lors de l'analyse du filtrat prélevé, et
- les moyens de détermination sont reliés au premier circuit entre lesdits moyens de prélèvement et ladite première vanne par un deuxième circuit comportant une deuxième vanne fermée lors du prélèvement et ouverte lors de l'analyse du filtrat prélevé.

Le dispositif selon l'invention peut en particulier être utilisé pour la détermination de la concentration, la masse et/ou la quantité d'au moins un soluté extraite lors du traitement et aussi sa concentration dans le filtrat et par calcul, celle dans le sang.

L'invention porte également sur un appareil de traitement extracorporel du sang mettant en oeuvre un dispositif de caractérisation d'un traitement extracorporel du sang selon l'invention.

D'autres avantages et caractéristiques de l'invention apparaîtront à l'examen de la description détaillée d'un mode de mise en oeuvre nullement limitatif, et des dessins annexés sur lesquels :

- les figures 1 et 2 sont des représentations schématiques d'un premier mode de réalisation d'un dispositif selon l'invention ;
- les figures 3 à 6 sont des représentations schématiques d'un deuxième mode de réalisation d'un dispositif selon l'invention ;
- la figure 7 est une représentation schématique d'un appareil selon l'invention intégrant un dispositif selon l'invention ; et
- la figure 8 est une représentation d'un appareil selon l'invention.

Sur les figures les éléments communs à plusieurs figures conservent la même référence.

Les exemples non limitatifs décrits dans la suite de la description concernent la caractérisation d'une dialyse.

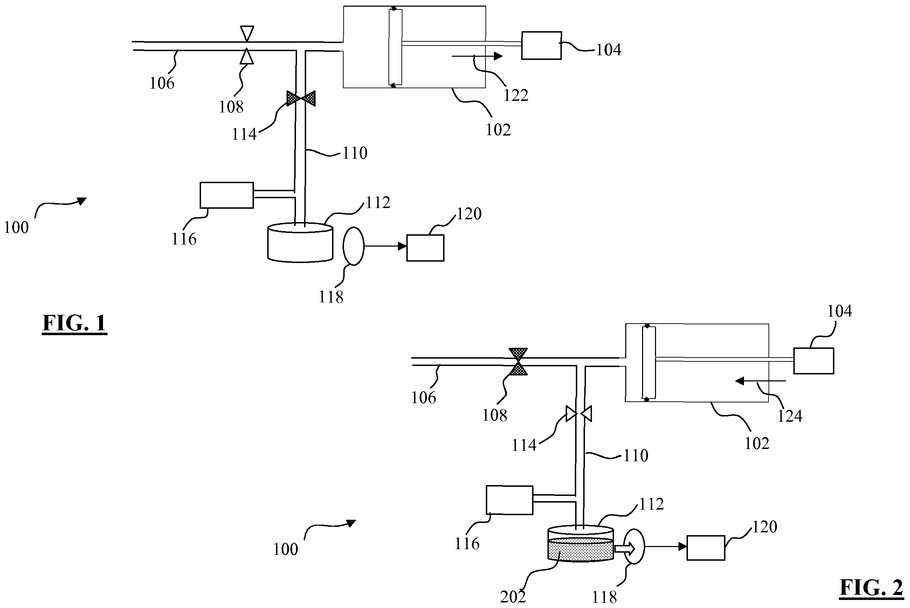

Les figures 1 et 2 sont des représentations schématiques d'un dispositif 100 selon l'invention selon un premier mode de réalisation.

La figure 1 est une représentation du dispositif 100 lors d'une phase de prélèvement continue du filtrat, qui dans le présent exemple est du dialysat usé et la figure 2 est une représentation du dispositif lors d'une phase de détermination d'un paramètre dans le dialysat usé prélevé.

Le dispositif 100 comporte une seringue 102, dite de prélèvement continu, associée à un premier moteur 104 permettant d'actionner cette seringue de prélèvement continu dans les deux sens. La seringue 102 est montée sur un circuit 106, dite de prélèvement continu, relié au circuit d'évacuation du dialysat usé (non représenté).

Une vanne 108, dite de prélèvement, est disposée sur ce circuit de prélèvement continu 106.

Un conduit 110, dit de mesure, est relié au circuit de prélèvement continu 106 entre la vanne 108 et la seringue 102. Ce conduit 110 débouche sur un réceptacle de mélange transparent 112. Une vanne, dite de mesure 114, est disposée sur ce conduit de mesure 110 avant le réceptacle de mélange 112. Un réservoir 116 comprenant un ou plusieurs réactifs et/ou d'indicateurs est connecté à ce conduit de mesure 110 après la vanne de mesure 114.

Le dispositif 100 comprend en outre un photomètre 118 disposé en vis-à-vis du réceptacle de mélange 112. Le photomètre 118 est relié à un module de calcul 120.

Nous allons maintenant décrire le fonctionnement du dispositif 100 en référence aux figures 1 et 2.

Sur la figure 1, le dispositif 100 réalise le prélèvement en continu d'une partie du dialysat usé lors de la dialyse. La vanne de prélèvement 108 est donc ouverte et la vanne de mesure 114 est fermée. Le moteur 104 déplace le piston de la seringue 102, de manière continue et à vitesse constante dans le sens du remplissage de la seringue 102 symbolisé par la flèche 122 sur la figure 1. La seringue 102 se remplit de manière continue lors de la dialyse.

Une fois la dialyse terminée, le dispositif 100 est en configuration de mesure représentée sur la figure 2. La vanne de prélèvement 108 est fermée et la vanne de mesure 114 est ouverte. Le moteur 104 déplace le piston de la seringue 102 dans le sens du vidage de la seringue 102 symbolisé par la flèche 124 sur la figure 2. La seringue 102 se vide dans le réceptacle de mélange 112 au travers du conduit de mesure 110. Un ou plusieurs réactif(s) et/ou indicateur(s) sont également versé(s) dans le réceptacle de mélange 112 depuis le réservoir 116.

Pour analyser plusieurs paramètres du même prélèvement. Les réactifs et indicateurs seront versés alternativement pour chaque dosage. Le réservoir 116 sera rempli pour au moins un réactif et/ou au moins un indicateur pour un premier dosage. Une fois versés ce réactif/indicateur versé(s), le réservoir 116 sera rincé et sera rempli pour le dosage suivant.

Le dialysat, le réactif et/ou l'indicateur se mélangent dans le réceptacle de mélange 112 et le résultat du mélange est une solution liquide colorée dont la coloration est mesurée par le photomètre 118. Le résultat de la mesure est transmis au module de calcul 120 qui détermine par estimation la valeur du paramètre recherché.

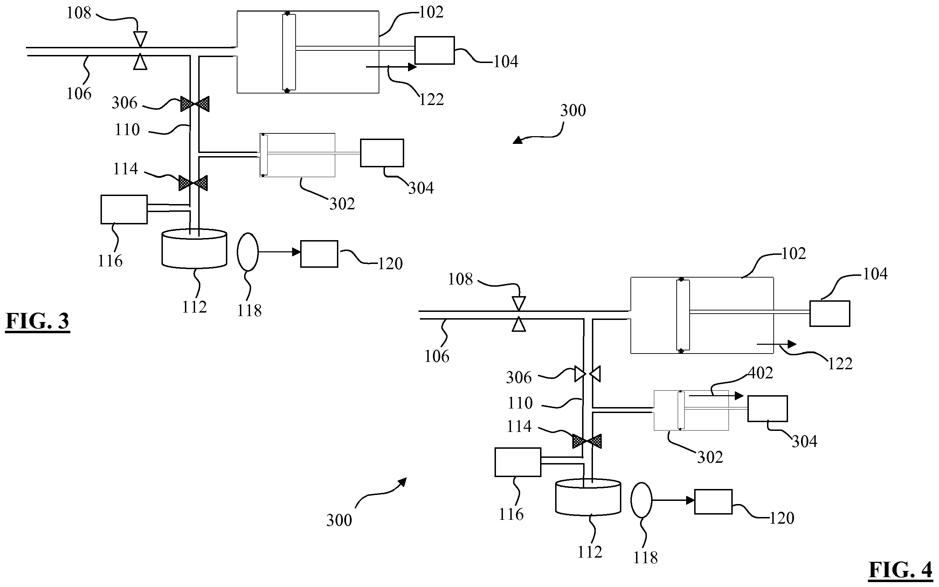

Les figures 3 à 6 sont des représentations schématiques d'un deuxième mode de réalisation d'un dispositif selon l'invention.

Le dispositif 300 représenté sur les figures 3 à 6 comporte tous les éléments du dispositif 100 représenté sur les figures 1 et 2. En plus de ces éléments le dispositif 300 comporte

Une seringue 302, dite d'échantillonnage instantané, reliée au conduit de mesure 110 avant la vanne de mesure 114. La seringue d'échantillonnage instantané 302 est associée à un moteur 304 permettant d'actionner le piston de cette seringue 302 dans les deux sens. Une vanne 306, dite d'échantillonnage instantané, est disposée sur le conduit de mesure 110 entre le seringue d'échantillonnage instantané 302 et le circuit de prélèvement 106.

Grâce à la seringue d'échantillonnage instantané 302, le dispositif 300 permet de réaliser un prélèvement instantané d'un échantillon du dialysat usé à un instant t lors de la dialyse pour déterminer la valeur instantanée d'un paramètre recherché à l'instant t.

Nous allons maintenant décrire le fonctionnement du dispositif 300 en référence aux figures 3 à 6.

La figure 3 est une représentation schématique du dispositif 300 en configuration de prélèvement continue. Dans cette configuration, la vanne de prélèvement 108 disposée sur le circuit de prélèvement continu 106 est ouverte. La vanne de mesure 114 et la vanne d'échantillonnage instantané 306 disposées sur le conduit de mesure 110 sont fermées. Le moteur 104 de la seringue de prélèvement continu 102 actionne le piston de cette seringue 102 dans le sens du remplissage de la seringue 102, symbolisé par la flèche 122, à une vitesse constante. La seringue de prélèvement continu 102 se remplit de manière continue.

La figure 4 est une représentation schématique du dispositif 300 en configuration d'échantillonnage instantané. Dans cette configuration, la seringue de prélèvement continu 102 continue à se remplir de la même manière que dans la configuration décrite en référence à la figure 3 et la vanne de mesure 114 est toujours fermée. Cependant, à l'instant t, la vanne d'échantillonnage instantané 306 est ouverte. Le moteur 304 associée à la seringue d'échantillonnage instantané 302 déplace le piston de cette seringue 302 dans le sens du remplissage de symbolisé par la flèche 402. La seringue d'échantillonnage instantané 302 se remplit d'un échantillon du dialysat usé à l'instant t. Le dialysat usé a donc été échantillonné à l'instant t.

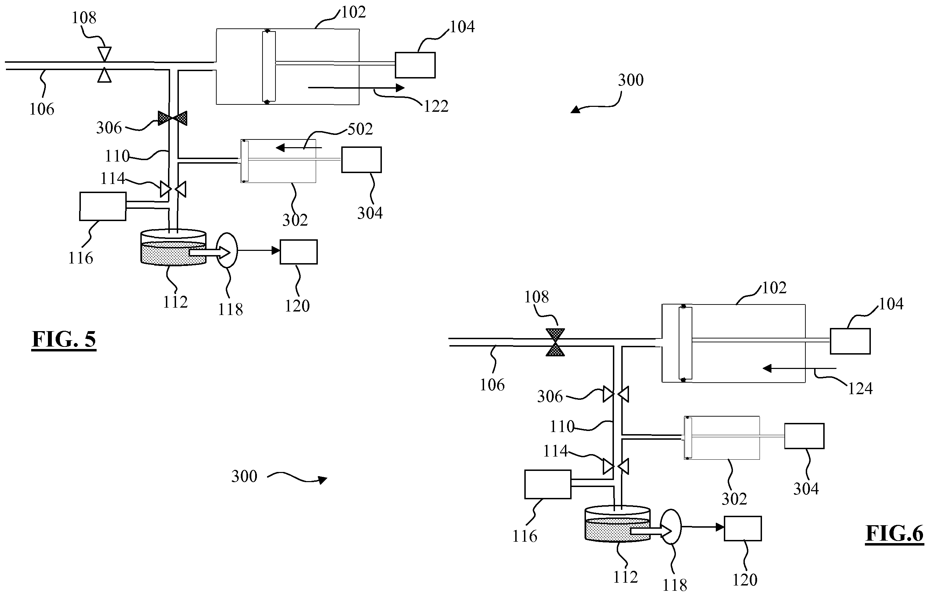

La figure 5 est une représentation schématique du dispositif 300 en configuration de mesure de la valeur instantanée d'un paramètre. La vanne de prélèvement continu 108 est toujours ouverte et la seringue de prélèvement continu 102 continue à se remplir sous l'action du moteur 104. Cependant, après le prélèvement de l'échantillon instantané à l'instant t, la vanne d'échantillonnage instantané 306 est fermée. La vanne de mesure 114 est ouverte et le moteur 304 actionne le piston de la seringue d'échantillonnage instantané 302 dans le sens du vidage de cette seringue 302 symbolisé par la flèche 502. L'échantillon prélevé à l'instant t, est donc vidé dans le réceptacle de mélange 112 au travers du conduit de mesure 110. Un ou plusieurs réactif(s) et/ou indicateur(s) sont également versé(s) dans le réceptacle de mélange 112 depuis le réservoir 116.

L'échantillon, le réactif et/ou l'indicateur se mélangent dans le réceptacle de mélange 112 et le résultat du mélange est une solution liquide colorée dont la coloration est mesurée parle photomètre 118. Le résultat de la mesure est transmis au module de calcul 120 qui détermine par estimation la valeur instantanée du paramètre recherché à l'instant t. Après la mesure le réceptacle de mélange 112 est vidé et nettoyé et la vanne de mesure 114 est fermée. Le prélèvement continu du dialysat usé dans la seringue de prélèvement continu 102 continue jusqu'à la fin de la dialyse.

Une fois la dialyse terminée, le dispositif 300 est en configuration de mesure représentée sur la figure 6. La vanne de prélèvement 108 est fermée et la vanne de prélèvement instantanée 306 et la vanne de mesure 114 sont ouvertes. Le moteur 304 de la seringue d'échantillonnage instantané n'est pas actif. Le moteur 104 déplace le piston de la seringue de prélèvement continu 102 dans le sens du vidage de la seringue 102 symbolisé par la flèche 124 sur la figure 6. La seringue 102 se vide dans le réceptacle de mélange 112 au travers du conduit de mesure 110. Un ou plusieurs réactif(s) et/ou indicateur(s) sont également versé(s) dans le réceptacle de mélange 112 depuis le réservoir 116.

Le dialysat usé, le réactif et/ou l'indicateur se mélangent dans le réceptacle de mélange 112 et le résultat du mélange est une solution liquide colorée dont la coloration est mesurée par le photomètre 118. Le résultat de la mesure est transmis au module de calcul 120 qui détermine par estimation la valeur moyenne sur toute la durée de la dialyse du (des) paramètre(s) recherché(s).

Le réservoir 116 est un réservoir de plusieurs réactif(s) et/ou indicateur(s). Chaque réactif et/ou indicateur peut être stocké dans une ou plusieurs seringues actionnées par un moteur ou dans un ou plusieurs contenants, tels que des flacons, actionnés par une pompe, un piston ou tout autre moyen.

Chaque réactif peut être transporté de la seringue contenant le réactif au réceptacle de mélange 112, soit par un circuit au moins en partie commun à tous les réactifs, soit par un circuit séparé, dédié et utilisé uniquement pour ce réactif. Dans ce dernier cas, il existe autant de circuits que de réactifs entre le réservoir 116 et le réceptacle 112.

Le dispositif selon l'invention peut en outre comprendre un module de commande (non représenté) comprenant des moyens informatiques et réalisant la commande des différents moteurs 104 et 304, et des différentes vannes 108, 114 et 306 ainsi que la commande des différents moteur du réservoir 116, du photomètre et du module de calcul.

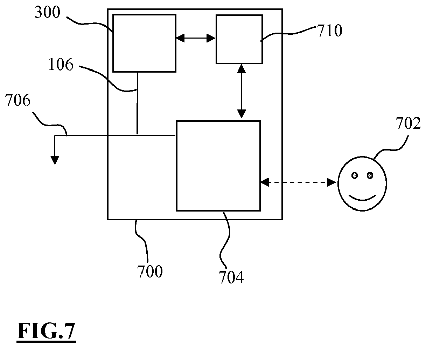

La figure 7 est une représentation schématique d'un appareil de dialyse selon l'invention intégrant un dispositif selon l'invention.

L'appareil de dialyse 700 est relié au patient 702 et réalise une dialyse du patient 702 grâce à un module de dialyse 704 comprenant par exemple une membrane semi-perméable (non représenté). Le dialysat usé est évacué vers un conduit d'eaux usées grâce à un circuit d'évacuation 706.

L'appareil de dialyse 700 peut en outre comprendre un module 710 récupérant du module de dialyse 704 des données relatives entre autres :

- au démarrage et à l'arrêt de la dialyse,
- au flux de dialysat,
- à la dialysance qui est définie comme étant la quantité de soluté extraite du sang par unité de temps divisée par la différence de concentration entre le sang et le dialysat à l'entrée du module de dialyse,
- au temps de dialyse,
- au flux d'ultrafiltration,
- au poids du patient 702, et
- au mode de dialyse réalisé par le module de dialyse.

Ce module 704 peut être connecté au dispositif 300 et fournir une partie de ces données au dispositif 300 et plus particulièrement au module de commande du dispositif 300. Ainsi, le module de commande peut réaliser une commande automatique :

- du démarrage du prélèvement continu en même temps que le démarrage de la dialyse,
- de l'arrêt du prélèvement continu en même temps que l'arrêt de la dialyse,
- d'un échantillonnage à un instant t lors de la dialyse, et
- des différents moteurs, vannes ou mesures.

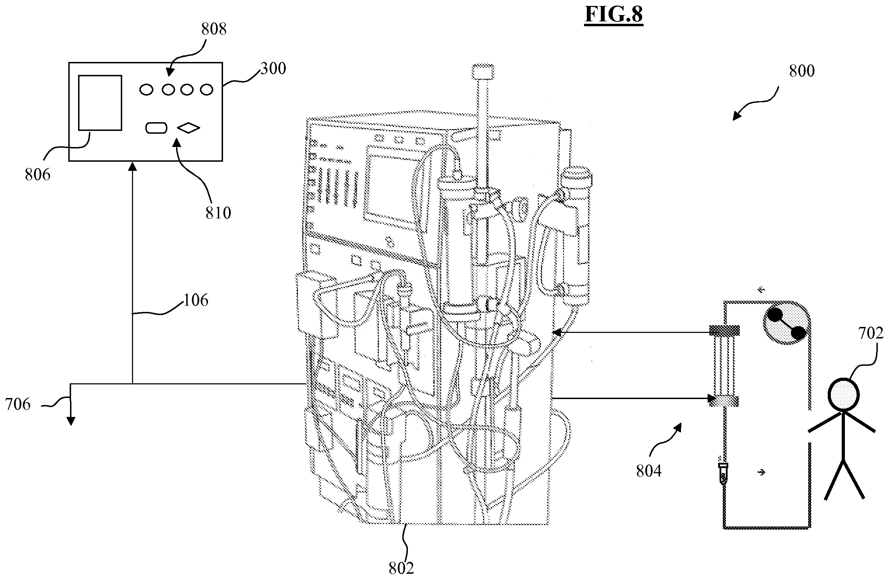

La figure 8 est une représentation schématique d'un appareil de dialyse selon l'invention dans lequel le dispositif selon l'invention n'est pas intégré.

L'appareil de dialyse 800 comprend donc un appareil de dialyse 802 qui est relié au patient 702 grâce à un circuit de dialyse 804 et réalise une dialyse du patient 702. Le dialysat usé est évacué vers un conduit d'eaux usées par un circuit d'évacuation 706 sur lequel est connecté le circuit de prélèvement continu 106 d'un dispositif 300 de caractérisation de la dialyse réalisée. L'appareil 300 comporte un écran 806 permettant de visualiser les valeurs des paramètres mesurés, des boutons de commande 808 et des ports de connexion 810 avec un appareil externe.

L'invention peut être mise en oeuvre pour :

- une mesure des taux de solutés, par exemple le taux de potassium, d'urée ou de créatinine ; de calcium ou encore de phosphate.
- un calcul de la masse totale extraite pour chaque soluté ;
- un calcul des concentrations estimées d'urée dans le sang du patient (valeurs : moyenne, avant et après séance) ;
- un calcul du taux de catabolisme des protéines (PCR) ; et/ou
- le calcul de tout autre paramètre calculé à partir des taux de solutés déterminés.

Les réactifs et indicateurs utilisés pour déterminer ces paramètres peuvent être :

- l'uréase et l'indophénol pour le taux d'urée prélevée, et

- soude, phosphate de sodium et acide picrique pour la créatinine.

Les réactifs utilisés sont souvent commercialisés sous forme de kit dont la composition est gardée secrète par le fabricant.

Nous allons maintenant décrire des exemples de calculs réalisés par le module de calcul pour la détermination de certains paramètres

**Calcul des masses extraites** :

Pour un soluté donné :

$$\text{Masse} = \text{concentration mesurée (C)} \times (\text{débit dialysat(Qd)} \times \text{temps séance(T)}$$
$$+ \text{ perte de poids sur la séance (WL))}$$

Voir Argiles, A, Ficheux, A, Thomas, A, et al: Precise quantification of dialysis using continuous sampling of spent dialysate and total dialysate volume measurement. Kidney Int 1997 52: 530-537

Pour l'urée on a :

$$Murée = Cdurée*(Qd * T + WL)$$

Pour le potassium on a :

$$Mpotassium = Cpotassium*(Qd * T + WL)$$

**Calcul des concentrations estimées d'urée dans le sang du patient (valeurs : moyenne, avant et après séance) :**

- Concentration moyenne estimée de l'urée plasmatique
$$= eCbm = Murée / Durée * T$$

La valeur de la dialysance (Durée) est nécessaire pour calculer la valeur moyenne estimée de l'urée. (eCbm). Cette valeur doit être indiquée manuellement ou être fournie par le générateur de dialyse.

- Concentration estimée d'urée plasmatique avant la dialyse
$$= Cbpre = eCbm* Durée*T/V / (1- exp (-Durée.T/V) )$$

- Concentration estimée d'urée plasmatique après la dialyse
$$= Cbpost = Cbpre* exp (-Durée.T/V)$$

La valeur du volume de distribution de l'urée chez le patient (V) est nécessaire pour calculer les concentrations estimées avant (Cbpre) et après séance (Cbpost). Cette valeur doit être indiquée manuellement ou calculée à partir de données anthropométriques du patient ou fournie par un appareil de mesure par bioimpédance du volume d'eau dans le corps :

*Exemple de formule anthropométrique : la formule de Watson et al :*

$$VWAT \text{ pour les hommes} = 2,447- 0,09516*age + 0,1074* hauteur + 0,3362* poids$$

$$VWAT \text{ pour les femmes} = 2,097+0,1069* hauteur +0,2466* poids$$

**Calcul du taux normalisé de catabolisme des protéines (nPCRw) :**

La formule de Garred et al pourra être utilisée pour ce calcul :

$$NPCRw = facteur 1; 2 ou 3 [Murée 1; 2 ou 3/BW] + 0.17$$

Les facteurs 1 (2.45), 2 (2.89) et 3 (3.10) dépendent du jour de la dialyse par rapport à la semaine et BW est l'objectif de poids à atteindre en fin de séance (appelé : poids sec).

Dans les exemples décrits le volume de la seringue de prélèvement continu 102 est d'environ 0.050 litre et le volume de la seringue d'échantillonnage instantané 302 est d'environ 0,002 litre.

Bien sûr, l'invention n'est pas limitée aux exemples qui viennent d'être décrits et de nombreux aménagements peuvent être apportés à ces exemples sans sortir du cadre de l'invention.

**Revendications**

1. Dispositif pour la caractérisation d'un traitement extracorporel du sang réalisé par un appareil de traitement extracorporel (704, 802) rejetant, lors dudit traitement, un liquide de traitement extracorporel, dit filtrat, évacué par un circuit d'évacuation (706), ledit dispositif comprenant :

   - des moyens (102, 104), dit de prélèvement, configurés pour constituer un réservoir de filtrat sur toute la durée dudit traitement, par prélèvement en continu, tout au long dudit traitement, d'une partie seulement dudit filtrat, en sortie dudit appareil de traitement (704, 802) ; et
   - des moyens (112, 116, 118, 120), dits de détermination, pour déterminer au moins un paramètre relatif audit traitement par analyse d'au moins un échantillon dudit réservoir de filtrat prélevé constitué par lesdits moyens de prélèvement (102, 104).

2. Dispositif selon la revendication 1, **caractérisé en ce que** les moyens de détermination comprennent des moyens (112) pour mélanger au moins une partie dudit filtrat avec au moins un réactif et/ou au moins un indicateur.

3. Dispositif selon l'une quelconque des revendications précédentes, **caractérisé en ce qu'**il comprend en outre des moyens d'échantillonnage (302, 304) pour prélever une quantité prédéterminée de filtrat, dit échantillon instantané, à un instant donné lors du traitement extracorporel.

4. Dispositif selon la revendication 3, **caractérisé en ce que** les moyens d'échantillonnage (302, 304) sont reliés aux moyens de détermination (112, 116, 118, 120), lesdits moyens de détermination (112, 116, 118, 120) réalisant la détermination d'une valeur instantanée, audit instant donné, d'au moins un paramètre par analyse dudit échantillon instantané de filtrat.

5. Dispositif selon les revendications 2 et 3, **caractérisé en ce que** les moyens de mélange comprennent, au moins en partie, les moyens d'échantillonnage.

6. Dispositif selon l'une quelconque des revendications précédentes, **caractérisé en ce que** les moyens de prélèvement (102,104) sont agencés pour prélever un échantillon inférieur à 50 ml de filtrat sur toute la durée du traitement.

7. Dispositif selon l'une quelconque des revendications précédentes, **caractérisé en ce que** les moyens de prélèvement (102, 104) sont reliés au circuit d'évacuation (706) par un premier circuit (106) comportant au moins une vanne (108), ladite vanne (108) étant :

   - ouverte lors du prélèvement, et
   - fermée lors de l'analyse du filtrat prélevé.

8. Dispositif selon l'une quelconque des revendications précédentes, **caractérisé en ce que** les moyens de prélèvement (102, 104) comprennent une seringue (102) reliée, au moins indirectement, au circuit d'évacuation (706) et actionnée de manière continue pour prélever le filtrat à un débit sensiblement constant depuis ledit circuit d'évacuation (706).

9. Dispositif selon l'une quelconque des revendications 3 à 8, **caractérisé en ce que** les moyens d'échantillonnage (302, 304) comprennent une seringue (302) reliée au circuit d'évacuation (706).

10. Dispositif selon l'une quelconque des revendications 8 ou 9, **caractérisé en ce qu'**il comprend au moins un moteur (104, 304) pour actionner une seringue (102, 302).

11. Dispositif selon l'une quelconque des revendications précédentes, **caractérisé en ce que** les moyens de détermination (112, 116, 118, 120) comprennent un photomètre (118).

12. Dispositif selon l'une quelconque des revendications précédentes, **caractérisé en ce que** :

- les moyens de prélèvement (102, 104) sont reliés au conduit d'évacuation (706) par un premier circuit (106) comportant une première vanne (108) ouverte lors du prélèvement et fermée lors de l'analyse du filtrat prélevé,
- les moyens de détermination (112, 116, 118, 120) sont reliés au premier circuit (106) entre lesdits moyens de prélèvement (102, 104) et ladite première vanne (108) par un deuxième circuit (110) comportant une deuxième vanne (114) fermée lors du prélèvement et ouverte lors de l'analyse du filtrat prélevé.

13. Utilisation du dispositif (100, 300) selon l'une quelconque des revendications 4 à 12, pour la mesure d'une valeur instantanée d'au moins un paramètre au début ou à un instant t au cours de la séance de traitement, ladite valeur étant utilisée pour la détermination :

- d'une quantité de dialysat à utiliser lors d'une dialyse,
- d'une composition du dialysat,
- d'une durée du traitement, ou
- d'un débit sanguin de traitement.

14. Utilisation selon la revendication 13, **caractérisée en ce que** le paramètre est la concentration de potassium.

15. Utilisation du dispositif selon l'une quelconque des revendications 13 ou 14, **caractérisée en ce que** le paramètre relatif au traitement est la concentration et/ou la masse prélevée d'au moins un soluté.

16. Appareil (700, 800) de traitement extracorporel mettant en œuvre un dispositif (100, 300) selon l'une quelconque des revendications 1 à 12.

17. Appareil (700) selon la revendication 16, intégrant un dispositif selon l'une quelconque des revendications 1 à 12.

**Patentansprüche**

1. Vorrichtung zur Charakterisierung einer extrakorporalen Blutbehandlung, welche anhand eines Geräts für extrakorporale Behandlung (704, 802) erfolgt, das während der Behandlung eine extrakorporale Behandlungsflüssigkeit, das sogenannte Filtrat, abscheidet, welches über eine Anordnung von Ablassleitungen (706) abgeleitet wird, wobei die Vorrichtung umfasst:

- Mittel (102, 104) zur sogenannten Entnahme, die eingerichtet sind, ein Filtratreservoir während der gesamten Dauer der Behandlung zu bilden, durch kontinuierliche Entnahme, während der Behandlung, nur eines Teils des Filtrats, am Ausgang des Behandlungsgeräts (704, 802); und
- Mittel (112, 116, 118, 120) zur sogenannten Bestimmung, um mindestens einen die Behandlung betreffenden Parameter durch Untersuchung mindestens einer Probe des durch die Entnahmemittel (102, 104) gebildeten Filtratreservoirs zu bestimmen.

2. Vorrichtung nach Anspruch 1, **dadurch gekennzeichnet, dass** die Bestimmungsmittel Mittel (112) zur Vermischung mindestens eines Teils des Filtrats mit mindestens einem Reagens und/oder mindestens einem Anzeiger umfassen.

3. Vorrichtung nach einem der vorhergehenden Ansprüche, **dadurch gekennzeichnet, dass** sie außerdem Probenahmemittel (302, 304) umfasst, um eine vorbestimmte Filtratmenge, die sogenannte Momentanprobe, zu einem gegebenen Zeitpunkt während der extrakorporalen Behandlung zu entnehmen.

4. Vorrichtung nach Anspruch 3, **dadurch gekennzeichnet, dass** die Probenahmemittel (302, 304) mit den Bestimmungsmitteln (112, 116, 118, 120) verbunden sind, wobei die Bestimmungsmittel (112, 116, 118, 120) zu dem gegebenen Zeitpunkt die Bestimmung eines Momentanwerts mindestens eines Parameters durch Untersuchung der Momentanprobe des Filtrats ausführen.

5. Vorrichtung nach den Ansprüchen 2 und 3, **dadurch gekennzeichnet, dass** die Vermischungsmittel zumindest teilweise die Probenahmemittel umfassen.

6. Vorrichtung nach einem der vorhergehenden Ansprüche, **dadurch gekennzeichnet, dass** die Entnahmemittel (102, 104) derart angeordnet sind, um eine Probe des Filtrats kleiner als 50 ml während der gesamten Fortdauer der Behandlung zu entnehmen.

**7.** Vorrichtung nach einem der vorhergehenden Ansprüche, **dadurch gekennzeichnet, dass** die Entnahmemittel (102, 104) mit der Anordnung von Ablassleitungen (706) über eine erste Anordnung (106) verbunden sind, welche mindestens ein Ventil (108) umfasst, wobei das Ventil (108):

- bei der Entnahme offen und
- bei der Untersuchung des entnommenen Filtrats geschlossen

ist.

**8.** Vorrichtung nach einem der vorhergehenden Ansprüche, **dadurch gekennzeichnet, dass** die Entnahmemittel (102, 104) eine Spritze (102) umfassen, welche wenigstens indirekt mit der Anordnung von Ablassleitungen (706) verbunden ist und kontinuierlich angetrieben wird, um das Filtrat in einem im Wesentlichen gleichbleibenden Durchsatz von den Ablassleitungen (706) aus zu entnehmen.

**9.** Vorrichtung nach einem der vorhergehenden Ansprüche 3 bis 8, **dadurch gekennzeichnet, dass** die Probenahmemittel (302, 304) eine mit der Anordnung von Ablassleitungen (706) verbundene Spritze (302) umfassen.

**10.** Vorrichtung nach einem der vorhergehenden Ansprüche 8 oder 9, **dadurch gekennzeichnet, dass** sie mindestens einen Motor (104, 304) zum Antrieb einer Spritze (102, 302) umfasst.

**11.** Vorrichtung nach einem der vorhergehenden Ansprüche, **dadurch gekennzeichnet, dass** die Bestimmungsmittel (112, 116, 118, 120) ein Photometer (118) umfassen.

**12.** Vorrichtung nach einem der vorhergehenden Ansprüche, **dadurch gekennzeichnet, dass**:

- die Entnahmemittel (102, 104) mit der Anordnung von Ablassleitungen (706) über eine erste Anordnung (106) verbunden sind, ein erstes Ventil (108) umfassend, welches bei der Entnahme offen und bei der Untersuchung des entnommenen Filtrats geschlossen ist,
- die Bestimmungsmittel (112, 116, 118, 120) mit der ersten Anordnung (106) zwischen den Entnahmemitteln (102, 104) und dem ersten Ventil (108) über eine zweite Anordnung (110) verbunden sind, ein zweites Ventil (114) umfassend, welches bei der Entnahme geschlossen und bei der Untersuchung des entnommenen Filtrats offen ist.

**13.** Verwendung der Vorrichtung (100, 300) nach einem der vorhergehenden Ansprüche 4 bis 12 zur Messung eines Momentanwerts mindestens eines Parameters am Anfang oder zu einem Zeitpunkt t während der Behandlungssitzung, wobei dieser Wert für die Bestimmung :

- einer bei einer Dialyse zu verwendenden Dialysatmenge,
- einer Dialysatzusammensetzung,
- einer Behandlungsdauer, oder
- eines Blutflusses für die Behandlung

herangezogen wird.

**14.** Verwendung nach Anspruch 13, **dadurch gekennzeichnet, dass** es sich bei dem Parameter um die Kaliumkonzentration handelt.

**15.** Verwendung der Vorrichtung nach einem der Ansprüche 13 oder 14, **dadurch gekennzeichnet, dass** es sich bei dem der Behandlung betreffenden Parameter um die Konzentration und/oder um die entnommene Masse mindestens einer Lösung handelt.

**16.** Gerät (700, 800) zur extrakorporalen Behandlung, das eine Vorrichtung (100, 300) nach einem der Ansprüche 1 bis 12 einsetzt.

**17.** Gerät (700) nach Anspruch 16, in welchem eine Vorrichtung nach einem der Ansprüche 1 bis 12 integriert ist.

**EP 2 493 525 B2**

**Claims**

1. A device for characterizing an extracorporeal blood treatment carried out by an extracorporeal treatment apparatus (704, 802) expelling, during said treatment, an extracorporeal treatment liquid, called filtrate, discharged through a discharge circuit (706), said device comprising:

   - means (102, 104), called collecting means, configured to constitute a reservoir of filtrate throughout the entire duration of said treatment, by continuous collection, throughout said treatment, of only a part of the filtrate, at the outlet of said treatment apparatus (704, 802); and
   - means (112, 116, 118, 120), called determining means, for determining at least one parameter relating to said treatment by analysis of at least one sample of said reservoir of filtrate constituted by said collecting means (102, 104).

2. The device according to claim 1, **characterized in that** the determining means comprise means (112) for mixing at least a part of said filtrate with at least one reagent and/or at least one indicator.

3. The device according to any one of the preceding claims, **characterized in that** it also comprises sampling means (302, 304) for collecting a predetermined quantity of filtrate, called instantaneous sample, at a given point in time during the extracorporeal treatment.

4. The device according to claim 3, **characterized in that** the sampling means (302, 304) are connected to the determining means (112, 116, 118, 120), said determining means (112, 116, 118, 120) carrying out the determination of an instantaneous value at said given point in time, of at least one parameter by analysis of said instantaneous sample of filtrate.

5. The device according to claims 2 and 3, **characterized in that** the mixing means comprise, at least in part, the sampling means.

6. The device according to any one of the preceding claims, **characterized in that** the collecting means (102, 104) are arranged in order to collect a sample of less than 50 ml of filtrate throughout the duration of the treatment.

7. The device according to any one of the preceding claims, **characterized in that** the collecting means (102, 104) are connected to the discharge circuit (706) by a first line (106) comprising at least one valve (108), said valve (108) being:

   - open during the collecting, and
   - closed during the analysis of the filtrate collected.

8. The device according to any one of the preceding claims, **characterized in that** the collecting means (102, 104) comprise a syringe (102) connected, at least indirectly, to the discharge circuit (706) and continuously actuated in order to collect the filtrate from said discharge circuit at a substantially constant flow rate (706).

9. The device according to any one of claims 3 a 8, **characterized in that** the sampling means (302, 304) comprise a syringe (302) connected to the discharge circuit (706).

10. The device according to any one of claims 8 or 9, **characterized in that** it comprises at least one motor (104, 304) for actuating a syringe (102, 302).

11. The device according to any one of the preceding claims, **characterized in that** the determining means (112, 116, 118, 120) comprise a photometer (118).

12. The device according to any one of the preceding claims, **characterized in that**:

   - the collecting means (102, 104) are connected to the discharge circuit (706) by a first line (106) comprising a first valve (108) that is open during the collecting and closed during the analysis of the filtrate collected,
   - the determining means (112, 116, 118, 120) are connected to the first line (106) between said collecting means (102, 104) and said first valve (108) by a second line (110) comprising a second valve (114) that is closed during the collecting and open during the analysis of the filtrate collected.

13. The use of the device (100, 300) according to any one of claims 4 to 12, for measuring an instantaneous value of at least one parameter at the start or at a point in time t during the treatment session, said value being used for determining:

- a quantity of dialysate to be used during a dialysis treatment,
- a composition of the dialysate,
- a duration of the treatment, or
- a treatment blood flow rate.

14. The use according to claim 13, **characterized in that** the parameter is the concentration of potassium.

15. The use of the device according to any one of claims 13 or 14, **characterized in that** the parameter relating to the treatment is the concentration and/or the collected mass of at least one solute.

16. An apparatus (700, 800) for extracorporeal treatment utilizing a device (100, 300) according to any one of claims 1 to 12.

17. The apparatus (700) according to claim 16, incorporating a device according to any one of claims 1 to 12.

FIG. 1

FIG. 2

FIG. 4

FIG. 3

**FIG. 5**

**FIG.6**

EP 2 493 525 B2

**FIG.7**

FIG.8

EP 2 493 525 B2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ARGILES, A ; FICHEUX, A ; THOMAS, A et al.** Precise quantification of dialysis using Continuous Sampling of Spent Dialysate and total dialysate volume measurement. *Kidney Int,* 1997, vol. 52, 530-537

- **ARGILES, A ; FICHEUX, A ; THOMAS, A et al.** Precise quantification of dialysis using continuous sampling of spent dialysate and total dialysate volume measurement. *Kidney Int,* 1997, vol. 52, 530-537